# EUROPEAN PATENT SPECIFICATION

(11) **EP 1 231 873 B1**
(45) Date of publication and mention of the grant of the patent: **24.08.2005**
(21) Application number: 00977620.4
(22) Date of filing: 14.11.2000
(51) Int. Cl.: A61F 5/01

(54) **DEVICE FOR DROP FOOT**
VORRICHTUNG FÜR GELÄHMTEN FUSS
DISPOSITIF POUR PIED TOMBANT

(30) Priority: 16.11.1999 FI 992462
(43) Date of publication of application: 21.08.2002
(73) Proprietor: Tiikkala, Eero, 90580 Oulu (FI)
(72) Inventor: Tiikkala, Eero, 90580 Oulu (FI)
(74) Representative: Laurinolli, Tapio Kullervo
(86) International application number: PCT/FI2000/000991
(87) International publication number: WO 2001/035876

(56) References cited:
- DE-U- 1 970 630
- US-A- 4 651 723

## Description

The invention is related to a device for drop foot and especially to this kind of device which includes a longitudinal curved spring for supporting the ankle of the drop foot, the spring being attached to the front part of the leg and to the foot in the area of the instep, respectively.

Especially with spinal cord injuries, a common condition resulting in is a disorder of the peroneus muscle (paresis peronei) which means that the ankle is without the function of lifting the foot, i.e. the foot is dropping. Walking without a device lifting the foot or keeping it at right angle is almost impossible for a person suffering from this disorder.

In the most simple devices for drop foot there is a stem part to be attached to the leg and another part for supporting the sole of the foot which parts keep the ankle at an approximately right angle. The stem part is placed on the back part or on the side of the leg. In some devices of this kind there is a resilient joint or a joint allowing a restricted movement placed in registration with the ankle joint. This kind of devices have many drawbacks. Stiff stem part and support part forced into a shoe are as such uncomfortable. They cause often abrasions on the foot. Because the part supporting the foot must fit the shape of the sole, it is also sweltering. In a solution, for avoiding these problems, a shoe is functioning as a support part and the heel thereof is provided with a sleeve part to which the stem part attached to the leg is connected. A drawback of this solution is that a specially made shoe is needed.

For avoiding these drawbacks, also solutions are developed in which the foot is supported with means, normally a spring arrangement, attached to the front part of the leg and to the foot in the area of the instep, respectively. In the solution of DE patent 327817 a curved spring is attached to the leg and the end of the spring adapted to the shape of the instep is set between the upper part of the shoe and the instep of the foot.

In US patent 4651723 one end of a plate like curved spring is attached slidably to the front part of the leg and the other end is placed between the upper part of a shoe and the instep of the foot. A drawback of these solutions is that the spring between the upper part of the shoe and the instep is rubbing the instep, although there may be a sock therebetween. Depending on materials of the shoe and the sock and the tightness of the fitting, sliding of the spring is very indefinite. In the solution of US patent 4651723, the sliding attachment to the front part of the leg increases the indefinitness of the operation of the device and reduces the supporting effect of the spring on the foot. Especially, the plate like spring of said US patent is moving indefinitely and uncontrollably also laterally between the upper part of the shoe and the instep of the foot, which means that the lateral support of the device on the foot is bad.

DE-U-1970630 presents a solution in which a spring or elastic strip is attached between the front part of the leg and the instep of the foot. In one embodiment an elastic band includes a guide for the spring at the front part of the leg or at the instep of the foot. The guide is formed by slits in the band. As the band is elastic, the lateral support of the ankle is not very good. As the band is pressed tightly against the spring, a lot of friction is caused and no easy sliding provided.

For alleviating the above problems, an object of the invention is to present an improvement to the solution in which the foot is supported with a longitudinal curved spring attached to the front part of the leg and to the foot in the area of the instep, respectively.

A device for drop foot including for supporting the foot a longitudinal curved spring, means for attaching the spring at a first end thereof to the front part of the leg (1), and means for attaching the spring at a second end thereof to the foot in the area of the instep including a guide for receiving the spring for gliding, is characterised in that the guide includes a counterpiece made of hard material, the counterpiece being adapted to receive the spring at the second end thereof tightly enough for lateral support of the ankle, and the counterpiece being adapted to receive the spring at the second end thereof with such a clearance and having such material properties that easy sliding of the spring in the longitudinal direction thereof is made possible.

In an embodiment of the invention the counterpiece is a sheath-shaped part adapted to be placed on the instep.

In another embodiment of the invention the counterpiece is attached to a shoe in the area of the instep.

The spring iss advantageously generally strip-shaped. The section of the spring may be curved or profiled otherwise for preventing it from twisting and so for improving lateral steadiness of the device.

The attachment of the spring at the first end thereof to the front part of the leg is advantageously adjustable in the longitudinal direction of the spring.

The solution according to the invention allows easy bending of the foot against the force of the spring during walking. At rest, the foot is returned easily to the basic position at essentially right angle to the leg. Abrasion of the foot, instep or leg is fully prevented. Shoes may be easily provided for the use of the device of the invention, and there will be no auxiliary parts inside the shoes uncomfortable for the foot. By standardising the solution formed by the spring and the counterpiece, the counterpiece may be easily attached to a shoe in the factory. As the spring is laterally quite tightly adapted to the counterpiece, the device provides good support for the ankle also laterally. The device according to the invention may also be made very lightweight.

The invention and some embodiments thereof are described in further detail in the following with reference to the accompanying drawings, in which:
Fig. 1 is a scematic side view, partially in section, presenting an embodiment of the device according to the invention as put in the place thereof;
Fig. 2 is a schematic perspective view presenting the device of Fig. 1;
Figs. 3 to 5 present schematically operation of an embodiment of the device according to the invention in connection with a shoe;
Figs. 6 to 10 are schematic sectional views presenting some possible realizations of the counterpiece and the spring in a solution according to the invention; and
Fig. 11 presents schematically some further embodiments of the invention.

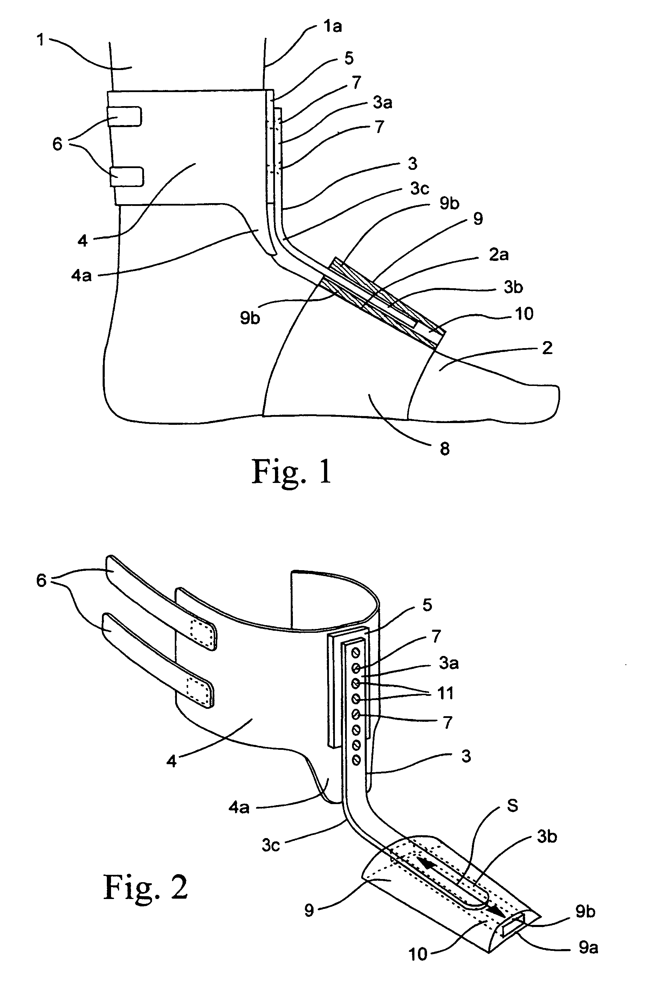

The device of Figs. 1 and 2 includes a conventional band 4 for attachment to the leg 1. Straps 6 and corresponding places on the band are provided with velcro tabs, for example, so that the band may be easily adjusted according to the thickness of the leg and may be tightened suitably. The front part 4a of the band 4 is extended to protect the ankle from possible pressing of the spring 3 against the ankle. Advantageous in the device according to the invention in comparison with some other devices is that the most suitable position of attachment of the band to the leg is the lowest part of the leg just above the ankle which is normally the thinnest part of the leg where the band is properly keeping the position thereof without strong tightening.

A plate 5, made of e.g. suitable metal, is attached to the band on the front part of the leg for attachment of the spring 3. At the upper end 3a of the spring there are several holes 11 close to each other through which the attachment to the plate may be made with screws 7. By selecting the attachment holes the vertical position of the spring may be adjusted suitably for the leg of a user. The proper adjustment of the vertical position of the spring is important for proper operation of the device. For steadiness of the device, it is advantageous that the attachment at the upper end of the spring is fixed. On the other hand, also alternatives in which there is certain clearance in the attachment, according to a user's desire or for a certain use, are within the scope of the invention. For example, the attachment may be slidable within certain limits.

The adjustable attachment described here is only exemplary. Equivalent adjustable attachments are known in many applications, and any suitable and strong enough attachment may be used also in the device according to the invention.

The spring 3 is curved so that it generally follows the contour of the front part of the leg end the instep as the ankle is in the normal position thereof during standing, i.e. at approximately right angle like in Fig. 1. The section of the first end 3a intended to be attached to the front part of the leg and correspondingly the section of the second end 3b intended to be attached to the instep may be essentially straight (Fig. 1). Such a design of the spring and the attachments is the most simple, but within the scope of the invention also such realizations are possible in which the spring 3 and the corresponding counterparts for the attachments are to some extent curved. The curved section 3c of the spring which in the examples presented here is simply following the contour of the ankle may be shaped also so that it goes at a little greater distance from the ankle for avoiding possible pressing of the spring against the ankle as the leg is bent towards the foot. In the examples of Figs. 1 and 2, the spring is a strip with a rectangular section and rounded second end 3b. A suitable material for making a spring is spring steel but also other materials, like fiber reinforced plastics or composite materials, may be suitable. If a spring is made of steel, for example, it may be advantageous to cover or protect it with insulating material at the intermediate section thereof where it goes close to the ankle.

For attachment in the area of the instep 2a, the device of Figs. 1 and 2 includes a counterpiece 9 for the spring 3b. The counterpiece 9 is a sheath-shaped piece the lower surface of which is conformed to the shape of the instep and the upper surface of which is curved so that the section of the piece is like a narrow crescent. Advantageously, the piece 9 is also to some extent tapered towards the toes. Such a design of the counterpiece makes it as imperceptible as possible as attached to a shoe. A channel 10 going through the counterpiece is dimensioned for the spring 3b with a suitable clearance so that the spring is sliding easily within it as indicated by arrow S (Fig. 2). The channel 10 must not necessarily extend through the piece, as it does in the figures, but a channel extending through the piece is the most easy to make. The part 9a of the counterpiece between the instep 2a and the spring 3b prevents abrasion between the spring 3b and the instep 2a. On the other hand, the force of the spring 3 affects mostly the part 9b of the counterpiece above the spring, and the structure of the counterpiece must be strong enough so that it endures the spring force and remarkable variations thereof in the use, for example during walking.

The most simple is to make the counterpiece of suitable, hard enough and strong plastic against which the spring is sliding well. Polyethene, for example, may be a suitable material. The counterpiece may be made of suitable metal, composite material or even ceramic material, too.

The force of the spring must be large enough so that it keeps the ankle easily in the normal rectangular position and returns it easily thereto. Experience from experimental devices indicates also that it is recommendable that the spring is to some extent tensioned, i.e. is lifting the foot already in the normal position of the ankle.

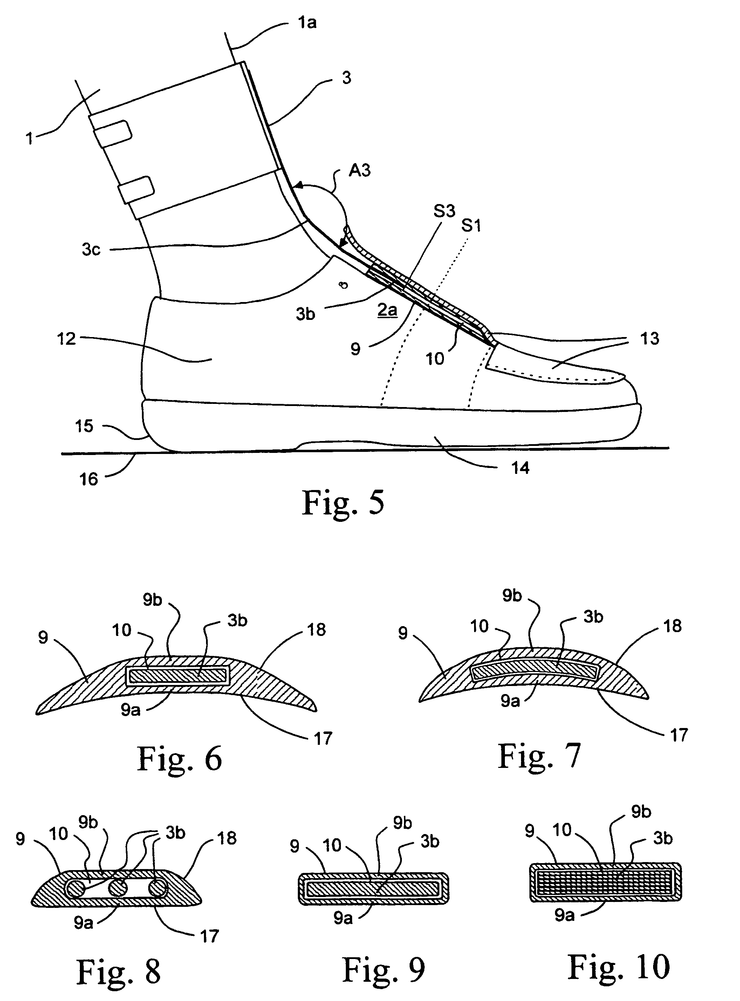

Figs. 6 to 10 present sectional views of some embodiments of the spring 3b and the counterpiece 9. Reference signs 9a and 9b indicate parts coming against the spring therebelow and thereabove, respectively. Reference signs 17 and 18 indicate the shaped lower surface and the upper surface of the counterpieces, respectively, in Figs. 6 to 8. In the examples of Figs. 6 to 8, the counterpiece 9 is an integral piece made of plastic, for example, including the channel 10 for a spring. In the example of Fig. 6, the spring 3b and the channel 10 are essentially rectangular in section. In the example of Fig. 7, the spring 3b and the channel 10 are curved in section. In the example of Fig. 8, the spring 3b is formed by three parallel wire-shaped parts. In the examples of Figs. 9 and 10, the channel 10 and the counterpiece 9 are formed by a tube 10 which is rectangular in section and made of steel, for example, for as good endurance as possible against the wear caused by the spring 3b. In the example of Fig. 10, the spring 3b is formed by several stacked plate springs.

The relatively narrow counterpiece of Figs. 9 and 10 with even surface and even thickness and with a width of about 20 millimeters, for example, is well suited for installation to a shoe. Obviously, the counterpiece may be realized in many other ways, too. It is not necessary that the counterpiece forms an integral channel for the spring, but it may be formed by only guides around the edges of the spring, for example.

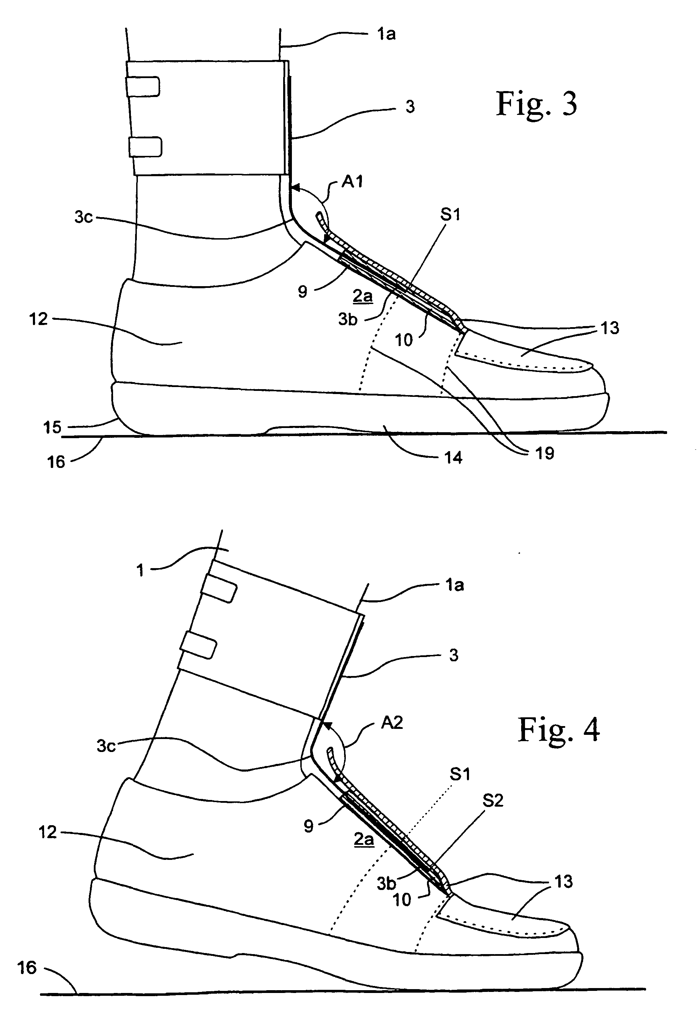

The counterpiece 9 may be attached to the foot 2 by means of a band or equivalent, for example, as is shown schematically in Fig. 1. Preferably, however, the device according to the invention is used in connection with a shoe. A shoe may be any suitable indoor or outdoor shoe. In the example of Figs. 3 to 5, the counterpiece is placed under the upper part 13 covering the instep in a moccasine type shoe 12. In Fig. 3, broken line 19 indicates a reinforcement made in the shoe for better endurance of the use of the device. The spring 3 affects mostly by lifting the counterpiece 9 and tends to turn it upwards. So, the structure of the shoe and the attachments of the counterpiece must be strong enough to endure this stress. In a band shoe, the counter piece may be attached to the tongue of the shoe whereby it is to be attached by the bands under the upper part. Also a bag of leather or fabric may be sewed on a shoe for keeping the counterpiece in place. It may be contemplated also that suitable holes are made at the edges of a counterpiece through which holes it may be sewed to a shoe or equivalent. Also gluing may be used for attachment. The device according to the invention may be used also at night to keep the ankle in a bent position. This is important because drop foot otherwise easily results in shortening of the Achilles tendon. Then, the counterpiece may be attached to a suitable band or even to a felt shoe which is stiff and durable enough.

As the spring 3 is made of spring steel, the thickness thereof may be about 1.5 millimeters, for example. The width of the spring may be about 15 millimeters, for example. The width of the spring may be reduced especially within the curving section 3c to about 6 millimeters, for example. The thickness of the counterpiece may be about 5 millimeters, and if desired, it may be made even a little thinner by selecting suitable materials. The length of the counterpiece may be 50 to 60 millimeters, for example. The clearance between the spring and the counterpiece must be large enough so that the spring is sliding easily. A suitable clearance is of the order of 0.3 to 0.5 millimeters, for example.

Figs. 3 to 5 illustrate operation of the device according to the invention during walking and a shoe according to the invention. The attachment of the spring 3 to the front part of the leg is according to that described in Figs. 1 and 2, for example, and is described only very schematically in Figs. 3 to 5. The shoe 12 is of moccasine type having a rounded heel 15 and sturdy sole 14. The ground is indicated by reference sign 16. The counterpiece 9 is placed under the upper part covering the instep typical to this kind of shoes. In this case, it may be easiest to attach the counterpiece to the lower surface of the upper part. In a normal approximately rectangular position (Fig. 3) the curving angle at the section 3c is A1, and the end 3b of the spring extend to point S1 in the channel 10 of the counterpiece 9.

As the foot is left behind during walking (Fig. 4), the angle A2 at the section 3c is sharper and the force of the spring is now directed through the counterpiece 9 to the instep 2a. The end 3b of the spring is moving forward in the channle 10 of the counterpiece 9 to point S2. Easy sliding of the spring in the counterpiece makes possible this change of the angle which makes walking easier. As the foot is raised forward again, the spring returns the angle of the ankle to normal.

As the foot is moved forward and put down to the ground (Fig. 5), the force directed by teh ground 16 to the heel 15 of the shoe turns the foot forward. The angle A3 at the section 3c of the spring is now larger, and the end 3b of the spring is sliding in the channel 10 of the counterpiece 9 backwards to point S3. The force of the spring turns now the foot back towards the normal position which make walking easier as the foot is again moving backwards.

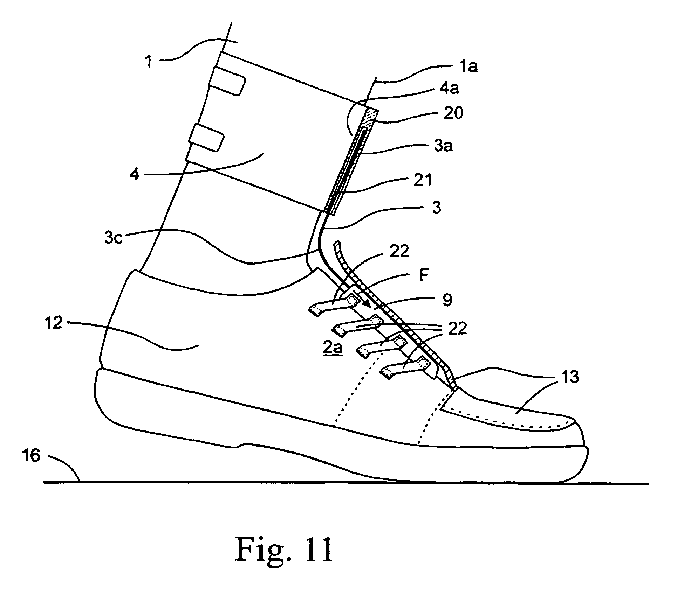

Furthermore, the attachment of the counterpiece 9 to the shoe in the area of the instep 2a may be also flexible. In Fig. 11, the attachment with flexible bands 22 is presented schematically, the ends of the bands being attached to the shoe and to the counterpiece 9, respectively. Then, the counterpiece 9 may yield a little as indicated by arrow F as the ankle is bent to a sharp angle. This makes walking easier if e.g. the spring for some reason or other is sliding poorly in the counterpiece.

In Fig. 11, also the attachment of the upper end 3a of the spring to the leg 1 is presented schematically in which attachment a part 20 is attached to the front side 4a of the band 4, the part 20 having a sheath-like space 21 for the upper end 3a of the spring. In the sheath-like space 21 there are one or more friction surfaces, and the end 3a of the spring is suitably roughened or coated for friction attachment. This kind of attachment makes changing of the spring easier, for example.

The counterpiece of the spring, which is an essential feature of the invention, is easy to attach to various shoes. With certain shoes, the counterpiece may be attached already in a factory. On the other hand, one may have the counterpieces attached by a shoemaker, and in the most cases one is also able to self attach the counterpieces to shoes with a reasonable effort.

Some embodiments of the invention are described above in detail, and also some further alternatives are considered. The invention is by no means restricted to these embodiments and alternatives but may vary within the scope of the accompanying claims.

## Claims

1. A device for drop foot including for supporting the foot:
a longitudinal curved spring (3);
means (5, 7) for attaching the spring (3) at a first end (3a) thereof to the front part (1a) of the leg (1); and
means for attaching the spring (3) at a second end (3b) thereof to the foot (2) in the area of the instep (2a) including a guide for receiving the spring (3) for gliding; **characterised in that** the guide includes a counterpiece (9, 10) made of hard material and adapted to receive the spring (3) at the second end (3b) thereof tightly enough for lateral support of the spring and thereby the ankle and with such a clearance that easy sliding of the spring in the longitudinal direction (S) thereof is made possible.

2. A device according to claim 1, **characterised in that** the clearance is of the order of 0.3 to 0.5 millimeters.

3. A device according to claim 1, **characterised in that** the counterpiece is a sheath-shaped part (9, 10, 17, 18) adapted to be placed on the instep.

4. A device according to claim 1, **characterised in that** the counterpiece (9, 10) is attached to a shoe (12, 13) in the area of the instep (2a).

5. A device according to claim 1, **characterised in that** the spring (3) is generally strip-shaped.

6. A device according to claim 4, **characterised in that** the section of the spring (3) is curved or profiled otherwise for preventing twisting thereof and for improving thereby lateral steadiness of the device.

7. A device according to claim 1, **characterised in that** in the means for attaching the spring (3) at the first end (3a) thereof to the front part (1a) of the leg (1) the attachment (5, 7, 11) is adjustable in the longitudinal direction of the spring (3).

## Patentansprüche

1. Die Vorrichtung für gelähmten Fuß enthält folgende Teile zum Stützen des Fußes:
längliche gebogene Feder (3),
Mittel (5, 7) zum Befestigen der Feder (3) am oberen Ende (3a) auf der Vorderseite (1a) des Beins (1) und
Mittel zum Verbinden der Feder (3) am unteren Ende (3b) im Bereich des Spanns (2a) mit dem Fuß (2) einschließlich einer Führung zum Empfangen der Feder (3) fürs Gleiten,
**dadurch gekennzeichnet, dass** die Führung ein Gegenstück (9, 10) enthält, das aus hartem Material hergestellt ist und angepasst worden ist, die Feder (3) am anderen Ende (3b) zu empfangen, um fest genug die Feder und damit den gelähmten Fuß seitlich zu stützen, und mit einem solchen Zwischenraum vorgesehen ist, dass ein leichtes Gleiten der Feder in der Längsrichtung (S) ermöglicht ist.

2. Vorrichtung gemäß Anspruch 1, **dadurch gekennzeichnet, dass** der Zwischenraum 0,3-0,5 Millimeter beträgt.

3. Vorrichtung gemäß Anspruch 1, **dadurch gekennzeichnet, dass** das Gegenstück ein scheidenförmiges Teil (9, 10, 17, 18) ist, das angepasst ist, um auf den Spann zu platzieren.

4. Vorrichtung gemäß Anspruch 1, **dadurch gekennzeichnet, dass** das Gegenstück (9, 10) am Schuh (12, 13) im Bereich des Spanns (2a) befestigt ist.

5. Vorrichtung gemäß Anspruch 1, **dadurch gekennzeichnet, dass** die allgemeinde Form der Feder (3) streifenförmig ist.

6. Vorrichtung gemäß Anspruch 5, **dadurch gekennzeichnet, dass** der Querschnitt der Feder (3) bogenförmig oder sonst profiliert ist, um das Sichdrehen zu verhindern und **dadurch** die seitliche Stabilität der Vorrichtung zu erhöhen.

7. Vorrichtung gemäß Anspruch 1, **dadurch gekennzeichnet, dass** die Mittel zum Befestigen der Feder (3) am oberen Ende (3a) auf der Vorderseite (1a) des Beins (1) die Befestigung (5, 7, 11) in der Längsrichtung der Feder (3) einstellbar ist.

## Revendications

1. Dispositif pour pied tombant incluant dans le but de soutenir le pied:
un ressort longitudinal courbé (3) ;
des moyens (5, 7) pour fixer le ressort (3) par sa première extrémité (3a) à la partie antérieure (1a) de la jambe (1) ; et
des moyens pour fixer le ressort (3) par sa deuxième extrémité (3b) sur la face dorsale (2a) du pied (2) ces moyens comprenant un guide recevant le ressort (3) pour le glissement de celui-ci ;
**caractérisé en ce que** le guide comprend une contrepartie (9, 10) faite d'un matériau dur et adaptée pour recevoir à son autre extrémité (3b) le ressort (3) de manière suffisamment serrée dans le but de soutenir dans le sens latéral le ressort et donc la cheville, et avec un jeu permettant au ressort de glisser aisément dans le sens longitudinal (S).

2. Dispositif selon la revendication 1, **caractérisé en ce que** le jeu est de l'ordre de 0,3 à 0,5 millimètres.

3. Dispositif selon la revendication 1, **caractérisé en ce que** la contrepartie est une pièce en forme de fourreau (9, 10, 17, 18) adaptée pour être placée sur la face dorsale du pied.

4. Dispositif selon la revendication 1, **caractérisé en ce que** la contrepartie (9,10) est fixée à la chaussure (12, 13) à l'emplacement de la partie dorsale du pied (2a).

5. Dispositif selon la revendication 1, **caractérisé en ce que** le ressort (3) est généralement en forme de lamelle.

6. Dispositif selon la revendication 5, **caractérisé en ce que** la section du ressort (3) est courbée ou autrement profilée afin d'empêcher son entortillement et d'améliorer ainsi la stabilité latérale du dispositif.

7. Dispositif selon la revendication 1, **caractérisé en ce que** dans les moyens pour fixer le ressort (3) par sa première extrémité (3a) à la partie antérieure (1a) de la jambe (1) la partie fixation (5, 7, 11) est réglable dans le sens longitudinal du ressort (3).
